# EUROPEAN PATENT APPLICATION

(11) **EP 4 527 443 A1**
(43) Date of publication of application: **26.03.2025**
(21) Application number: 23816031.1
(22) Date of filing: 29.05.2023
(51) Int. Cl.: A61N 1/32, A61N 1/36

(54) **ORAL MUSCLE TRAINING INSTRUMENT AND ORAL MUSCLE TRAINING METHOD**

(30) Priority: 30.05.2022 JP 2022088048
(71) Applicant: National University Corporation Kobe University, Kobe-shi, Hyogo 657-8501 (JP)
(72) Inventor: KATO, Hiroshi, Kobe-shi, Hyogo 657-8501 (JP); YASUDA, Takahiro, Kobe-shi, Hyogo 657-8501 (JP)
(74) Representative: Fleck, Hermann-Josef
(86) International application number: PCT/JP2023/019993
(87) International publication number: WO 2023/234274

(57) **Abstract**

Provided are an oral muscle training instrument, whereby it becomes possible to effectively stimulate a genioglossus muscle that is a deep muscle in the oral cavity. The oral muscle training instrument is provided with: a pair of electrodes that are arranged in the oral cavity; an electrode that is branched from one of the pair of electrodes and is arranged on the surface of the skin; and an electric circuit that distributes a medium frequency current between the electrodes. The pair of electrodes are placed on the tongue, and the electrode arranged on the surface of the skin is placed below the chin or in the front of the neck. It is possible that the pair of electrodes is placed on the tongue and the electrode arranged on the surface of the skin is placed on approximately the entire circumference of the neck.

## Description

### Technical Field

The present invention relates to a technique for training oral muscles including tongue muscles, especially a genioglossus muscle, for the purpose of medical treatment or prevention of Sleep Apnea Syndrome (SAS) or dysphagia.

### Background Art

In sleep apnea syndrome, especially Obstructive Sleep Apnea Syndrome (OSAS), fatty tissue is deposited on a pharyngeal portion, narrowing an airway and increasing the flow rate during inspiration. This is because, generally, the human skeleton does not significantly grow after the age of around 20, whereas the amount of muscle, fat, etc. continues to increase even after the 20s, resulting in a narrowing of the airway area. Moreover, with age and specific diseases, pharyngeal tonus (autonomy of the tongue during sleep) decreases, so that the tongue is sucked into the trachea during inspiration, obstructing the airway and resulting in apnea. As a result, in a supine position, a normal person sleeps so that their mouth is closed and their tongue is raised, whereas a patient with sleep apnea syndrome sleeps so that their mouth is opened and their tongue is lowered. In order to improve the autonomy of tongue with respect to these symptoms, training of the tongue muscles including the genioglossus muscle through electric stimulation, restoring the pharyngeal tonus through nervous stimulation, etc. have been considered as effective countermeasures. Moreover, training of the oral muscle is effective also in medical treatment or prevention of dysphagia.

As a device for training tongue muscles, such as a genioglossus muscle through electric stimulation, an oral muscle training device has been known in which electrodes are disposed inside and outside the oral cavity to apply a current to the electrodes (refer to Patent Literature 1). The oral muscle training device disclosed in Patent Literature 1 includes a plurality of electrode means and an electric circuit and is configured to apply a current to the electrode means to provide electric stimulation to the oral muscles, increasing a tension of the oral muscles and training the tongue muscles.

However, in the oral muscle training device disclosed in Patent Literature 1, there is a problem that two electrodes which are electrodes installed outside the oral cavity are disposed in a lateral direction of a midline of the user's face, which makes it difficult to effectively stimulate only targeted deep muscles (tongue muscles such as genioglossus muscle) in the oral cavity.

### Citation List

### Patent Literature

Patent Literature 1: Published Japanese Translation of PCT International Publication for Patent Application No. 2022-505209

### Summary of Invention

### Technical Problem

As described above, training of the tongue muscle through electric stimulation is effective for medical treatment or prevention of the sleep apnea syndrome, but conventional devices are not sufficiently capable of electrically stimulating the genioglossus muscle, which is the largest of the tongue muscles.

In the light of such a situation, the object of the present invention is to provide an oral muscle training device and an oral muscle training method, being capable of effectively stimulating a genioglossus muscle, which is a deep muscle in the oral cavity.

### Solution to Problem

In order to solve the above-described problem, an oral muscle training device according to a first aspect of the present invention includes: a pair of electrodes disposed in an oral cavity; an electrode branched from one electrode of the pair of electrodes and disposed on a skin surface; and an electric circuit configured to pass a medium frequency current between the electrodes. The deep muscle in the oral cavity can be effectively stimulated by disposing the pair of positive and negative electrodes in the oral cavity in a case of a direct current or the pair of electrodes in the case of alternating current, and branching one of the pair of electrodes as a skin electrode to be disposed. It is to be noted that the term skin surface used herein does not mean the entire surface of human skin surface, but means a skin surface below the tongue and above the heart. The purpose of limiting the skin surface to a position above the heart is to prevent electricity from flowing to the heart and causing arrhythmias.

In the oral muscle training device according to the first aspect of the present invention, it is preferable that the pair of electrodes is disposed on a tongue, and the electrode disposed on the skin surface is disposed under a chin or on a front surface of a neck. The pair of electrodes are disposed on the tongue and the electrode on the skin surface is disposed under the chin or the front surface of the neck, and thereby when the current is applied to the electrode, the current flows vertically along the human midline, more effectively stimulating the deep muscles in the oral cavity.

Alternatively, in the oral muscle training device according to the first aspect of the present invention, the pair of electrodes may be disposed on the tongue and the electrode disposed on the skin surface may be disposed around substantially an entire periphery of a neck. The electrode disposed on the skin surface being disposed around substantially the entire periphery of the neck, and thereby nerves and muscles of the genioglossus can be effectively stimulated. Moreover, a current density of the electrode disposed on the skin surface can be reduced, preventing blood flow in the carotid arteries from being inhibited.

In the oral muscle training device according to the first aspect of the present invention, the pair of electrodes may be respectively disposed at a tip of the tongue and a back of the tongue. It is to be noted that the disposition of the electrodes at the tip and back of the tongues defines a relative positional relationship of the pair of electrodes, and does not limit, for example, a distance between the electrodes. Moreover, the electrode having the equivalent electric potential to the electrode disposed on the skin surface may be the electrode disposed at the tip of the tongue or the electrode disposed at the back of the tongue.

In the oral muscle training device according to the first aspect of the present invention, the pair of electrodes may be disposed on a left side or a right side of a midline, and the electrode disposed on the skin surface may be disposed on an opposite side to the pair of electrodes across the midline.

Alternatively, in the oral muscle training device according to the first aspect of the present invention, the pair of electrodes may be disposed to be in point symmetry with respect to a point on a center line of the tongue between a tip of the tongue and a back of the tongue. It is to be noted that since the tongue tends to move unexpectedly, the term center line of the tongue and the term midline are distinctively used herein, but when the tongue is in a stationary state without moving to right or left, the term center line and the term midline are synonymous with each other.

Alternatively, in the oral muscle training device according to the first aspect of the present invention, the electrode disposed on the skin surface may be branched into at least two electrodes, and when there is an even number of branched electrodes, the electrodes may be disposed in line symmetry with respect to a midline, or when there is an odd number of branched electrodes, one electrode may be disposed on the midline and the rest of the electrodes may be disposed in line symmetry with respect to the midline.

In the oral muscle training device according to the first aspect of the present invention, as the pair of electrodes, there may be three electrodes respectively disposed at a tip of the tongue, a middle of the tongue, and a back of the tongue, and a pair may be formed of the electrodes disposed at the middle and tip of the tongue and another pair may be formed of the electrodes disposed at the middle and back of the tongue.

In the oral muscle training device of the first aspect of the invention, a plurality of pairs of electrodes may be disposed as the pair of electrodes, in which adjacent electrodes are opposite poles to each other.

In the oral muscle training device of the first aspect of the invention, the medium frequency current is a continuous wave current equal to or more than 3 kHz and less than 8 kHz. Alternatively, the medium frequency current may be an intermittent wave in which a current equal to or more than 3 kHz and less than 8 kHz flows continuously for a predetermined period of time and then does not flow for a predetermined period of time (e.g., one second or more), or a current equal to or more than 3 kHz and less than 8 kHz may be an intermittent wave current having a low frequency of 1 to 100 Hz (also referred to as a burst wave current in this specification). Additionally, the continuous wave current and the burst wave current may be combined with each other. The medium frequency current is more preferably equal to or more than 3 kHz and less than 5 kHz.

An oral muscle training device according to a second aspect of the present invention includes: a first pair of electrodes disposed in an oral cavity; a first surface electrode branched from one electrode of the first pair of electrodes and disposed on a skin surface; an electric circuit configured to pass a first medium frequency current between electrodes formed of the first pair of electrodes and the first surface electrode; a second pair of electrodes disposed in the oral cavity; a second surface electrode branched from one electrode of the second pair of electrodes and disposed on the skin surface; and an electric circuit configured to pass a second medium frequency current between electrodes formed of the second pair of electrodes and the second surface electrode.

In the oral muscle training device according to the second aspect of the present invention, it is preferable that the first and second pairs of electrodes are disposed on a tongue, and the first and second surface electrodes are disposed under a chin or on a front surface of a neck.

An electrode set according to the present invention is a set of the electrodes disposed in the oral cavity and the electrode disposed on the skin surface in the above-described any one of the oral muscle training devices.

An oral muscle training method according to the first aspect of the present invention includes each of the following steps 1) to 3):
1) disposing a pair of electrodes on a tongue in an oral cavity;
2) disposing an electrode branched from one electrode of the pair of electrodes on a skin surface under a chin or on a front surface of a neck; and
3) passing a medium frequency current between the electrodes.

In the oral muscle training method according to the first aspect of the present invention, the step of disposing the pair of electrodes may be disposing the pair of electrodes at a tip of the tongue and a back of the tongue.

In the oral muscle training method according to the first aspect of the present invention, the step of disposing the pair of electrodes may be disposing the pair of electrodes on a left side or right side of a midline, and the step of disposing on the skin surface may be disposing the electrode branched from the one electrode on an opposite side to the pair of electrodes across the midline.

In the oral muscle training method according the first aspect of the present invention, the step of disposing the pair of electrodes may be disposing the pair of electrodes to be in point symmetry with respect to a point on a center line of the tongue between the tip of the tongue and the back of the tongue.

In the oral muscle training method according to the first aspect of the present invention, the step of disposing on the skin surface may be branching the electrode branched from the one electrode into at least two, and when there is an even number of branched electrodes, may be disposing the electrodes in line symmetry with respect to the midline, or when there is an odd number of branched electrodes, may be disposing one electrode on the midline and may be disposing the rest of the electrodes in line symmetry with respect to the midline.

In the oral muscle training method according to the first aspect of the present invention, as the pair of electrodes, there may be three electrodes respectively disposed at a tip of the tongue, a middle of the tongue, and a back of the tongue, and a pair may be formed of the electrodes disposed at the middle and tip of the tongue and another pair may be formed of the electrodes disposed at the middle and back of the tongue.

Alternatively, the oral muscle training method according to the first aspect of the present invention may include each of the following steps 1) to 3):
1) disposing a pair of electrodes on a tongue in the oral cavity;
2) disposing an electrode branched from one electrode of the pair of electrodes around substantially an entire periphery of a neck; and
3) passing a medium frequency current between the electrodes.

An oral muscle training method according to the second aspect of the present invention includes each of the following steps A) to E):
A) disposing a first pair of electrodes on a tongue in an oral cavity;
B) disposing a second pair of electrodes on the tongue in the oral cavity;
C) disposing a first surface electrode branched from one electrode of the first pair of electrodes on a skin surface under a chin or on a front surface of a neck;
D) disposing a second surface electrode branched from one electrode of the second pair of electrodes on the skin surface under the chin or on the front surface of the neck; and
E) passing a first medium frequency current between electrodes formed of the first pair of electrodes and the first surface electrode and a second medium frequency current between electrodes formed of the second pair of electrodes and the second surface electrode.

### Advantageous Effects of Invention

According to the oral muscle training device and the oral muscle training method according to the present invention, it has an effect of making it possible to effectively stimulate the genioglossus muscle, which is the deep muscle in the oral cavity. Moreover, it also has an effect of making it possible to stimulate muscles needed for swallowing.

### Brief Description of Drawings

[Fig. 1] Fig. 1 is a functional block diagram of an oral muscle training device according to an embodiment 1.
[Fig. 2] Fig. 2 is a configuration image diagram of the oral muscle training device according to the embodiment 1.
[Fig. 3] Fig. 3 is a usage image diagram of the oral muscle training device according to the embodiment 1.
[Fig. 4] Fig. 4 is an image diagram of an electrode disposition of the embodiment 1.
[Fig. 5] Fig. 5 is a functional explanatory diagram of the oral muscle training device according to the embodiment 1.
[Fig. 6] Fig. 6 is an image diagram of an electrode disposition of an embodiment 2.
[Fig. 7] Fig. 7 is an image diagram of an electrode disposition of an embodiment 3.
[Fig. 8] Fig. 8 is an image diagram of an electrode disposition of an embodiment 4.
[Fig. 9] Fig. 9 is an image diagram of an electrode disposition of an embodiment 5.
[Fig. 10] Fig. 10 is an image diagram of an electrode disposition of an embodiment 6.
[Fig. 11] Fig. 11 is an image diagram of an electrode disposition of an embodiment 7.
[Fig. 12] Fig. 12 is an image diagram of an electrode disposition of an embodiment 8.
[Fig. 13] Fig. 13 is an image diagram of an electrode disposition of an embodiment 9.
[Fig. 14] Fig. 14 is an image diagram of an electrode disposition of an embodiment 10.
[Fig. 15] Fig. 15 is a functional block diagram of an oral muscle training device according to an embodiment 11.
[Fig. 16] Fig. 16 is a configuration image diagram of the oral muscle training device according to the embodiment 11.
[Fig. 17] Fig. 17 is an image diagram (1) of an electrode disposition of an embodiment 12.
[Fig. 18] Fig. 18 is an image diagram (2) of the electrode disposition of the embodiment 12.
[Fig. 19] Fig. 19 is a schematic flow chart of the oral muscle training method according to the embodiment 1.
[Fig. 20] Fig. 20 is a functional block diagram of an oral muscle training device according to an embodiment 12.
[Fig. 21] Fig. 21 is a configuration image diagram of the oral muscle training device according to the embodiment 12.
[Fig. 22] Fig. 22 is a schematic flow chart of an oral muscle training method according to the embodiment 12.
[Fig. 23] Fig. 23 is an image diagram of an electrode disposition of an embodiment 13.
[Fig. 24] Fig. 24 is a functional explanatory diagram of an oral muscle training device according to the embodiment 13.
[Fig. 25] Fig. 25 is an image diagram of an electrode disposition of an embodiment 14.
[Fig. 26] Fig. 26 is a functional explanatory diagram of an oral muscle training device according to the embodiment 14.

### Description of Embodiments

Hereinafter, an example of embodiments of the present invention will be described in detail with reference to the drawings. It is to be noted that the scope of the present invention is not limited to the following embodiments or illustrated examples, and that many modifications and variations can be envisaged.

### Embodiment 1

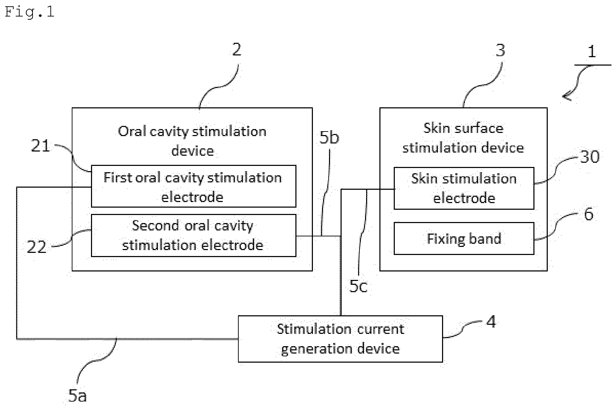

Fig. 1 shows a functional block diagram in one embodiment of an oral muscle training device according to the present invention. As shown in Fig. 1, the oral muscle training device 1 includes an oral cavity stimulation device 2, a skin surface stimulation device 3, and a stimulation current generation device 4.

The oral cavity stimulation device 2 includes a first oral cavity stimulation electrode 21 and a second oral cavity stimulation electrode 22 as a pair of electrodes disposed in an oral cavity. The stimulation current generation device 4 is connected to the first oral cavity stimulation electrode 21 through a lead wire 5a and is connected to the second oral cavity stimulation electrode 22 through a lead wire 5b.

The skin surface stimulation device 3 includes a skin stimulation electrode 30 and a fixing band 6. The fixing band 6 is provided with the skin stimulation electrode 30. The skin stimulation electrode 30 is an electrode branched from the second oral cavity stimulation electrode 22, which is one electrode of the pair of electrodes disposed in the oral cavity, and disposed on a skin surface, and is connected to the stimulation current generation device 4 through a lead wire 5c connected to the lead wire 5b.

The second oral cavity stimulation electrode 22 and the skin stimulation electrode 30 have an equivalent electric potential, and the second oral cavity stimulation electrode 22 and the skin stimulation electrode 30 have a different potential from the first oral cavity stimulation electrode 21.

The stimulation current generation device 4 is a device capable of applying a medium frequency current, and the medium frequency current is applied by operating operation buttons (4a, 4b) for switching the current ON/OFF and for adjusting a frequency of the medium frequency current. The medium frequency current is an alternating current having a frequency of 1 kHz to 10 kHz, but the present embodiment uses a continuous wave current (alternating current) having a frequency equal to or more than 3 kHz and less than 8 kHz. This is because, as a result of an experience experiment, electric stimulation is felt to be too strong in a case of being less than 3 kHz, while electric stimulation is not felt very well in a case of being equal to or more than 8 kHz.

Operation contents are displayed on a display unit 4c. The stimulation current generation device 4 and the lead wires (5a to 5c) form an electric circuit configured to pass the medium frequency current between the electrodes.

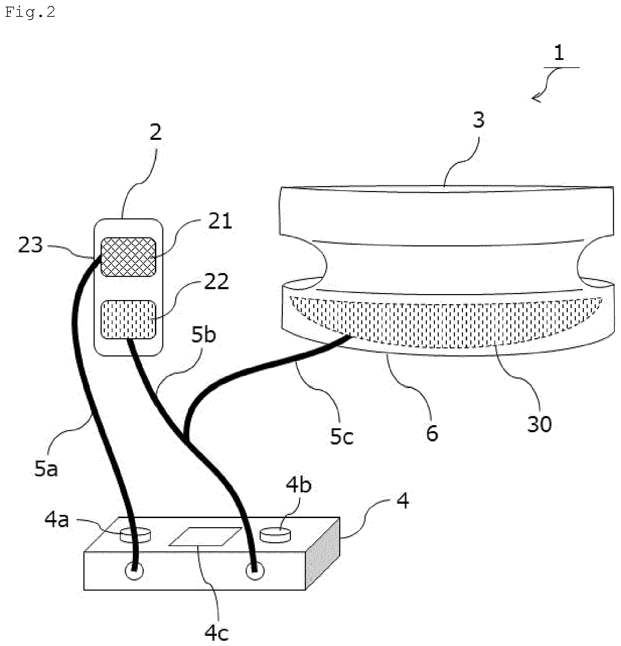

Fig. 2 shows a configuration image diagram of the oral muscle training device. As shown in Fig. 2, in the oral cavity stimulation device 2, the first oral cavity stimulation electrode 21 and the second oral cavity stimulation electrode 22 are provided on a base 23. The fixing band 6 is worn to cover a back of head to a chin of human and has the skin stimulation electrode 30 provided on an inner surface side thereof.

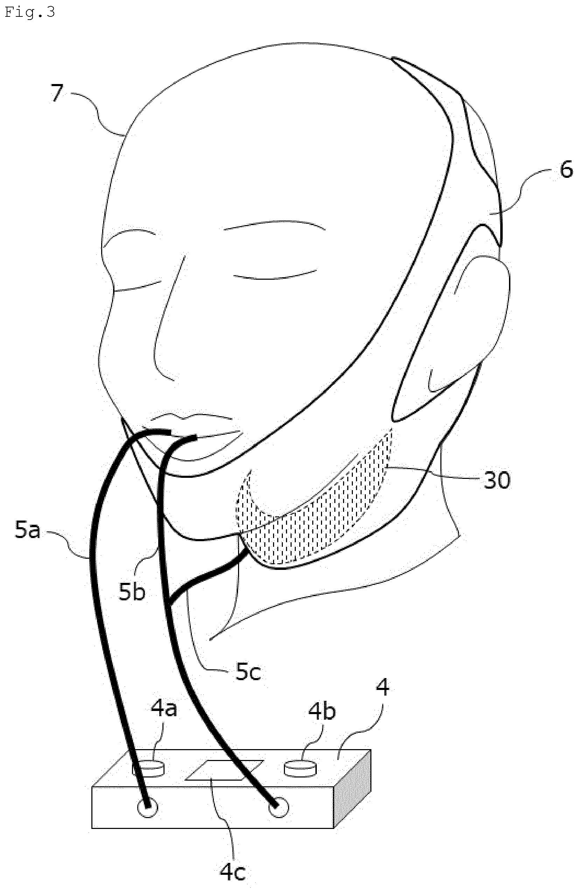

Fig. 3 shows a usage image diagram of the oral muscle training device. In use, as shown in Fig. 3, a user 7 wears the fixing band 6, to which the skin stimulation electrode 30 is attached, on the head and then holds the oral cavity stimulation device 2 shown in Fig. 2 in one's mouth. The skin stimulation electrode 30 is provided so as to extend right and left centered around a position in contact with a thyroid cartilage when the user 7 wears the fixing band 6. By fixing the skin stimulation electrode 30 to the fixing band 6, the user 7 can provide and fix the skin stimulation electrode 30 at an appropriate position merely by wearing the fixing band 6, thereby improving a training effect and convenience.

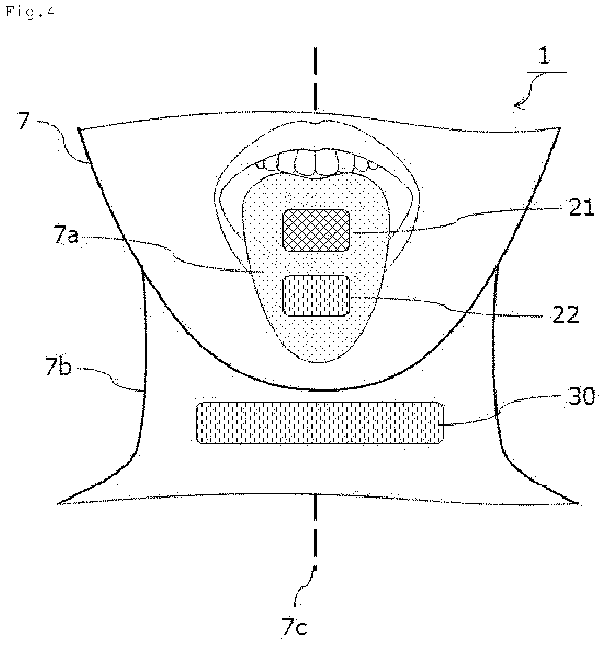

Fig. 4 shows an image diagram of electrode disposition in the present embodiment. It is to be noted that, for convenience of explanation, in Figs. 4, 6 to 14, 17, 18, and 23 to 26, only a disposition image of electrodes in the device is illustrated, and shapes of the electrode images are also deformed.

As shown in Fig. 4, in the oral muscle training device 1, the first oral cavity stimulation electrode 21 and the second oral cavity stimulation electrode 22 are disposed on a tongue 7a of the user 7 along a midline 7c, which is a line passing vertically straight through a center of the front surface of a human or animal; and the first oral cavity stimulation electrode 21 is disposed at a back of the tongue, i.e., on a throat side, and the second oral cavity stimulation electrode 22 is disposed at a tip of the tongue, i.e., on a mouth side.

Moreover, the skin stimulation electrode 30 is disposed so as to extend right and left centered around the midline 7c on the skin of a neck 7b of the user 7. By making the skin stimulation electrode 30 wide or longer in length, it is possible to pass the medium frequency current over a wider range to be stimulated. Furthermore, by using the skin stimulation electrode 30, the skin stimulation electrode can be formed of one electrode, allowing for a simple specification.

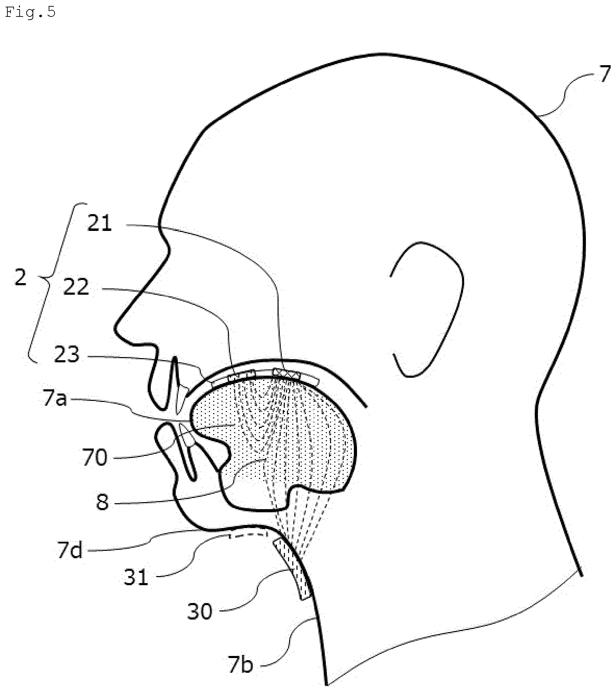

Fig. 5 shows a functional explanatory diagram of the oral muscle training device. As shown in Fig. 5, a positive or negative electrode is disposed on the tongue 7a of the user 7, and an electrode branched from the one electrode is disposed at a position of the neck, which is a position sandwiching oral muscles, such as a tongue muscle and a genioglossus muscle; and as shown in an image 8 of a current flow, the oral muscles such as the tongue muscle and the genioglossus muscle 70 are stimulated with a current flowing through the first oral cavity stimulation electrode 21, the second oral cavity stimulation electrode 22, and the skin stimulation electrode 30. The following Table 1 shows voltage, current, and resistance values of the oral cavity stimulation electrode or the skin stimulation electrode when the current of each frequency is applied. The applied current is a continuous wave current between 4 kHz to 8 kHz.

**[Table 1]**

| Frequency (Hz) | Oral cavity stimulation electrode | | | Skin stimulation electrode | | |
|---|---|---|---|---|---|---|
| | Voltage (V) | Current (mA) | Resistance (Ω) | Voltage (V) | Current (mA) | Resistance (Ω) |
| 4000Hz | 5.6 | **3.4** | 1765 | 6.9 | **10.8** | 639 |
| 6000Hz | 5.3 | **2.5** | 2240 | 5.2 | **13.0** | 400 |
| 8000Hz | 5.9 | **2.7** | 2185 | 5.2 | **13.4** | 385 |

As shown in Table 1, the current values flowing through the electrodes in the oral cavity are 3.4 mA at 4000 Hz, 2.5 mA at 6000 Hz, and 2.7 mA at 8000 Hz. In contrast, the current values flowing through the electrode on the skin of the neck are 10.8 mA at 4000 Hz, 13.0 mA at 6000 Hz, and 13.4 mA at 8000 Hz, indicating that the current flowing through the skin stimulation electrode is greater.

In this embodiment, the skin stimulation electrode 30 is disposed on the front surface of the neck 7b, but instead of this example, the skin stimulation electrode 31 as shown by the dashed line may be disposed at a position under the chin 7d. Moreover, the skin stimulation electrode 30 and the skin stimulation electrode 31 may have the same shape, or may be have different shapes from each other in accordance with human body shape. Furthermore, the skin stimulation electrode 30 and the skin stimulation electrode 31 may be used in combination.

By adopting such an electrode disposition, a current density within a range from the tongue to under the chin or the neck can be improved, and thereby oral muscles, such as the tongue muscle and the genioglossus muscle, can be effectively stimulated.

Embodiments 2 to 12 described below all show examples of disposing any one of skin stimulation electrodes (30, 30a to 30e) on the front surface of the neck 7b, but such an electrode may be disposed at a position under the chin 7d, as the skin stimulation electrode 31. Moreover, although not illustrated in the embodiments 2 to 10, a configuration of applying a current through a lead wire using the stimulation current generation device 4 is the same as that of the present embodiment.

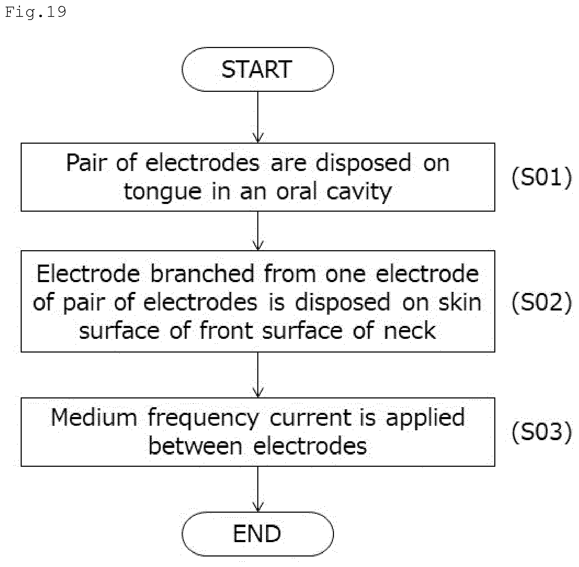

Fig. 19 shows a schematic flow chart of an oral muscle training method according to the present embodiment. As shown in Fig. 19, a pair of electrodes are first disposed on a tongue in an oral cavity (Step S01), and then an electrode branched from one electrode of the pair of electrodes is disposed on a skin surface of a front surface of a neck (Step S02). After such a disposition, a medium frequency current is applied between the electrodes (Step S03). It is to be noted that the order of Step S01 and Step S02 may be reversed.

### Embodiment 2

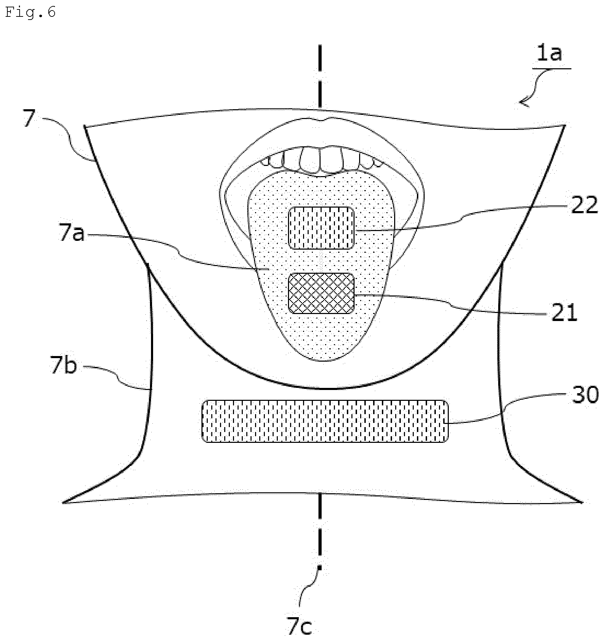

Fig. 6 shows an image diagram of electrode disposition according to another embodiment. As shown in Fig. 6, in an oral muscle training device 1a, a first oral cavity stimulation electrode 21 and a second oral cavity stimulation electrode 22 are disposed along a midline 7c on a tongue 7a of a user 7, i.e., the first oral cavity stimulation electrode 21 is disposed at a tip of the tongue, and the second oral cavity stimulation electrode 22 is disposed at a back of the tongue. Moreover, the skin stimulation electrode 30 is disposed so as to extend right and left centered around the midline 7c on the skin of a neck 7b of the user 7. The first oral cavity stimulation electrode 21 and the skin stimulation electrode 30 have opposite potentials to each other, and the second oral cavity stimulation electrode 22 and the skin stimulation electrode 30 have an equivalent electric potential. By adopting such an electrode disposition, the oral muscles at the tip side of the tongue can be trained more effectively when a current is applied to the electrodes.

### Embodiment 3

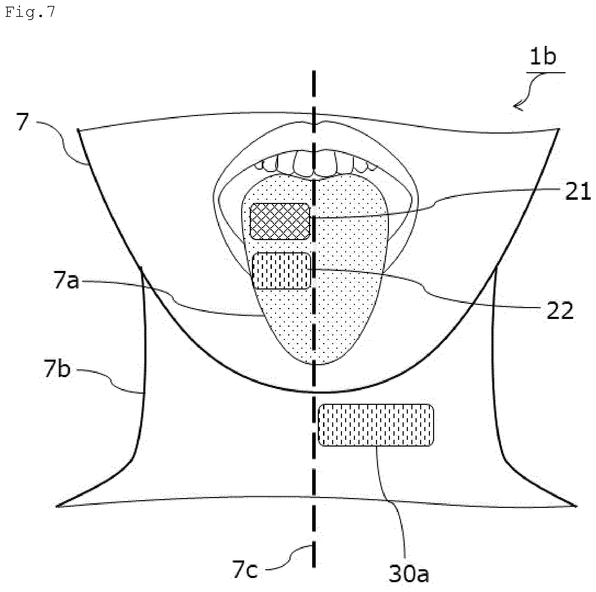

Fig. 7 shows an image diagram of electrode disposition according to another embodiment. As shown in Fig. 7, in an oral muscle training device 1b, a first oral cavity stimulation electrode 21 and a second oral cavity stimulation electrode 22 are disposed on a left side of a midline 7c on a tongue 7a of a user 7, i.e., the first oral cavity stimulation electrode 21 is disposed at a back of the tongue, and the second oral cavity stimulation electrode 22 is disposed at a tip of the tongue. Moreover, a skin stimulation electrode 30a is disposed on an opposite side to the first oral cavity stimulation electrode 21 and the second oral cavity stimulation electrode 22 across the midline 7c on a skin of a neck 7b of the user 7. The first oral cavity stimulation electrode 21 and the skin stimulation electrode 30a have opposite potentials to each other, and the second oral cavity stimulation electrode 22 and the skin stimulation electrode 30a have an equivalent electric potential.

It is to be noted that the disposition of the three electrodes may be reversed right and left, such that the first oral cavity stimulation electrode 21 and the second oral cavity stimulation electrode 22 are disposed on the right side of the midline 7c and the skin stimulation electrode 30a is disposed on the left side thereof. The oral cavity stimulation electrode 21 and the second oral cavity stimulation electrode 22 may be spaced apart by a predetermined distance, and their positions are not particularly important.

By adopting such an electrode disposition, even a single electrode under the chin or on the neck can selectively stimulate a center portion of a tongue muscle when the current is applied to the electrodes.

### Embodiment 4

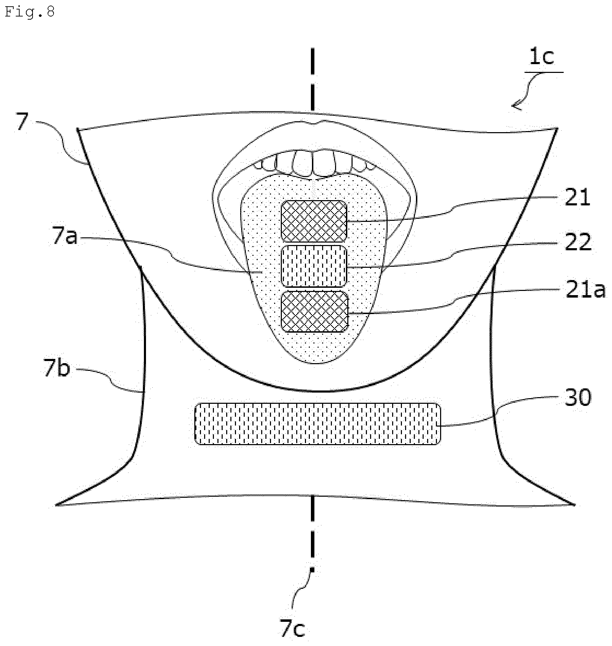

Fig. 8 shows an image diagram of electrode disposition according to another embodiment. As shown in Fig. 8, in the oral muscle training device 1c, first oral cavity stimulation electrodes (21, 21a) and a second oral cavity stimulation electrode 22 are disposed along a midline 7c on a tongue 7a of a user 7, i.e., the first oral cavity stimulation electrode 21 is disposed at a back of the tongue, the second oral cavity stimulation electrode 22 is disposed at the middle of the tongue, and the first oral cavity stimulation electrode 21a is disposed at a tip of the tongue. Thus, as the pair of electrodes, there are three electrodes respectively disposed at the tip of the tongue, the middle of the tongue, and the back of the tongue, and a pair is formed of the second oral cavity stimulation electrode 22 at the middle of the tongue and the first oral cavity stimulation electrodes (21, 21a) at the tip of the tongue and at the back of the tongue. Moreover, the skin stimulation electrode 30 is disposed so as to extend right and left centered around the midline 7c on the skin of a neck 7b of the user 7. The first oral cavity stimulation electrode 21 and the first oral cavity stimulation electrode 21a have an equivalent electric potential, and the second oral cavity stimulation electrode 22 and the skin stimulation electrode 30 have an equivalent electric potential. Additionally, the first oral cavity stimulation electrodes (21, 21a) and the second oral cavity stimulation electrode 22 have opposite potentials to each other, and the first oral cavity stimulation electrodes (21, 21a) and the skin stimulation electrode 30 have opposite potentials to each other.

Thus, three electrodes are provided on the tongue and the first oral cavity stimulation electrodes (21, 21a) are disposed so as to sandwich the second oral cavity stimulation electrode 22, and thereby a current density directly under the first oral cavity stimulation electrode 21 and the second oral cavity stimulation electrode 22 or the first oral cavity stimulation electrode 21a and the second oral cavity stimulation electrode 22 increases when applying the current to the electrodes, and therefore the oral muscles can be effectively trained.

### Embodiment 5

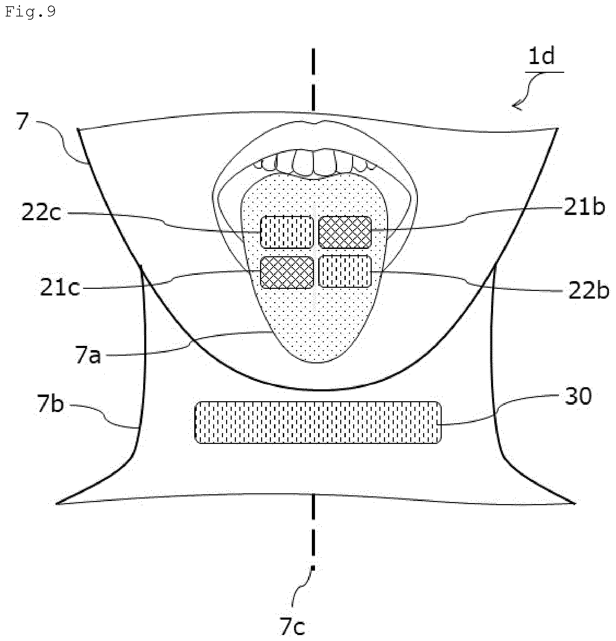

Fig. 9 shows an image diagram of electrode disposition according to another embodiment. As shown in Fig. 9, in an oral muscle training device 1d, first oral cavity stimulation electrodes (21b, 21c) and second oral cavity stimulation electrodes (22b, 22c) are disposed on a tongue 7a of a user 7. The first oral cavity stimulation electrode 21b and the first oral cavity stimulation electrode 21c have an equivalent electric potential, and the second oral cavity stimulation electrode 22b, the second oral cavity stimulation electrode 22c, and the skin stimulation electrode 30 have an equivalent electric potential. Moreover, the first oral cavity stimulation electrodes (21b, 21c) and the second oral cavity stimulation electrodes (22b, 22c) have opposite potentials to each other and the first oral cavity stimulation electrodes (21b, 21c) and the skin stimulation electrode 30 have opposite potentials to each other. Namely, the first oral cavity stimulation electrodes (21b, 21c) and the second oral cavity stimulation electrodes (22b, 22c) are disposed so that adjacent electrodes have opposite polarities to each other.

The pair of electrodes in the present embodiment is formed of the first oral cavity stimulation electrodes (21b, 21c), which are a plurality of electrodes, and the second oral cavity stimulation electrodes (22b, 22c), which are a plurality of electrodes, and the adjacent electrodes have opposite polarities to each other. Moreover, the skin stimulation electrode 30 is disposed so as to extend right and left centered around the midline 7c on the skin of a neck 7b of the user 7.

Thus, by disposing four electrodes on the tongue so that adjacent poles have opposite polarities to each other, a current density directly under the first oral cavity stimulation electrode 21b and the second oral cavity stimulation electrode 22b, the first oral cavity stimulation electrode 21b and the second oral cavity stimulation electrode 22c, the first oral cavity stimulation electrode 21c and the second oral cavity stimulation electrode 22b, or the first oral cavity stimulation electrode 21c and the second oral cavity stimulation electrode 22c increases when applying the current to the electrodes, and therefore the oral muscles can be effectively trained in a wider range corresponding to the four electrodes. Moreover, it is possible for only one electrode to be disposed under the chin or on the neck. By making the skin stimulation electrode width wide, it is possible to pass the current over a wide range to be stimulated.

### Embodiment 6

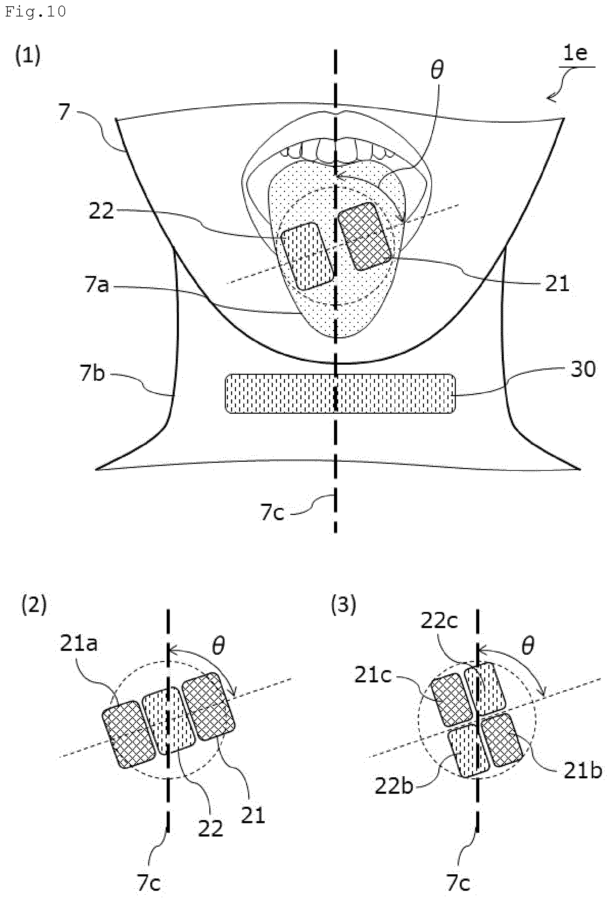

Fig. 10 is image diagram of electrode disposition in another embodiment, in which Fig. 10(1) shows a case of two electrodes, Fig. 10(2) shows a case of three electrodes, and Fig. 10(3) shows a case of four electrodes, each disposed on a tongue. An oral muscle training device 1e is obtained by rotating the electrode disposition state of any one of the oral muscle training devices (1, 1c, 1d) within a range of 180°. In the example shown in Fig. 10(1), a first oral cavity stimulation electrode 21 disposed at a back of the tongue and a second oral cavity stimulation electrode 22 disposed at a tip of the tongue is in a state of being rotated within a range of 180°. By setting an angle θ, an area under the tongue where a current density is high can be changed.

Moreover, in the example shown in Fig. 10(2), a first oral cavity stimulation electrode 21 disposed at a back of the tongue, a second oral cavity stimulation electrode 22 disposed at the middle of the tongue, and a first oral cavity stimulation electrode 21a disposed at a tip of the tongue are in a state of being rotated within a range of 180°. Moreover, in the example shown in Fig. 10(3), first oral cavity stimulation electrodes (21b, 21c) and second oral cavity stimulation electrodes (22b, 22c), which are disposed so that adjacent electrodes have opposite polarities to each other, are in a state of being rotated within a range of 180°. Also in this case, by setting an angle θ, an area under the tongue where a current density is high can be changed.

### Embodiment 7

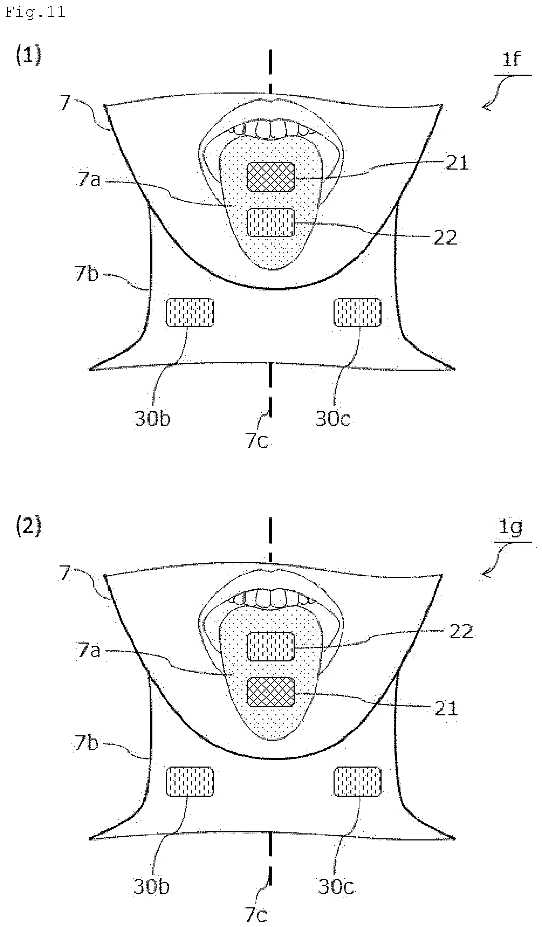

Fig. 11 is an image diagram of electrode disposition of another embodiment, for an electrode disposed on a skin surface that is branched into two electrodes, Fig. 11(1) shows a case where a first oral cavity stimulation electrode is disposed at a back of the tongue and a second oral cavity stimulation electrode is disposed at a tip of the tongue, and Fig. 11(2) shows a case where a first oral cavity stimulation electrode is disposed at a tip of the tongue and a second oral cavity stimulation electrode is disposed at a back of the tongue.

As shown in Fig. 11(1), in an oral muscle training device 1f, a first oral cavity stimulation electrode 21 and a second oral cavity stimulation electrode 22 are disposed along a midline 7c on a tongue 7a of a user 7, a second oral cavity stimulation electrode 22 is disposed at a tip of the tongue, and a first oral cavity stimulation electrode 21 is disposed at a back of the tongue. Moreover, skin stimulation electrodes (30b, 30c) are disposed on a skin of a neck 7b of the user 7 in line symmetry with respect to the midline 7c. As shown in Fig. 11(2), in an oral muscle training device 1g, a first oral cavity stimulation electrode 21 and a second oral cavity stimulation electrode 22 are disposed along a midline 7c on a tongue 7a of a user 7, and the first oral cavity stimulation electrode 21 is disposed at a tip of the tongue, and the second oral cavity stimulation electrode 22 is disposed at a back of the tongue. Moreover, skin stimulation electrodes (30b, 30c) are disposed on a skin of a neck 7b of the user 7 in line symmetry with respect to the midline 7c. Thus, in the oral muscle training device (1f, 1g), the skin stimulation electrodes (30b, 30c) are not disposed on the midline 7c, but are disposed in line symmetry with respect to the midline 7c, and therefore when the current is applied to the electrodes, the current flows vertically along the midline 7c of the user 7, effectively stimulating the deep muscles in the oral cavity. Such an advantageous effect also applies to embodiment 8 to 10 described below.

In any of the oral muscle training devices (1f, 1g), the second oral cavity stimulation electrode 22 and the skin stimulation electrodes (30b, 30c) have an equivalent electric potential. The first oral cavity stimulation electrode 21 and the second oral cavity stimulation electrode 22 have opposite potentials to each other, and the first oral cavity stimulation electrode 21, and the skin stimulation electrodes (30b, 30c) have opposite potentials to each other. By adopting such an electrode disposition, wide oral muscles can be selectively trained.

In the present embodiment, since there is an even number of electrodes disposed on the skin surface, the electrodes are disposed in line symmetry with respect to the midline 7c. In contrast, when there is an odd number of electrodes is disposed on the skin surface, e.g., when three electrodes are disposed on the skin of the neck 7b of the user 7, one electrode is disposed on the midline 7c, and the rest of the electrodes is disposed in line symmetry with respect to the midline 7c.

### Embodiment 8

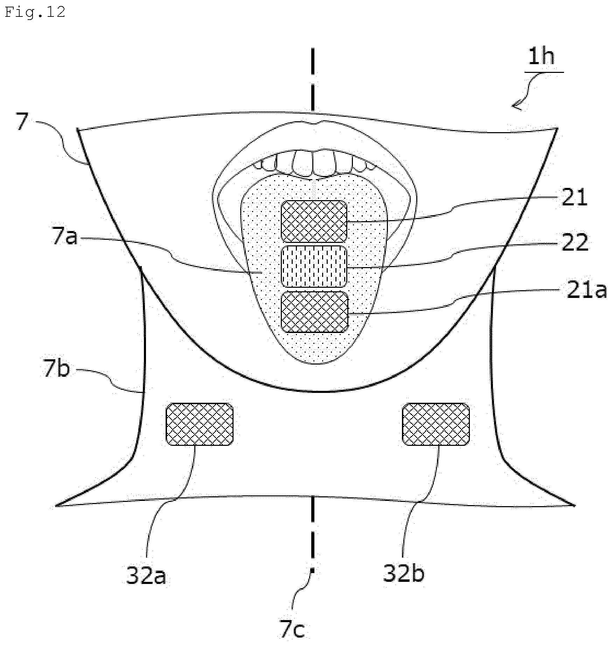

Fig. 12 shows an image diagram of electrode disposition according to another embodiment. As shown in Fig. 12, in an oral muscle training device 1h, a first oral cavity stimulation electrodes (21, 21a) and the second oral cavity stimulation electrode 22 are disposed along a midline 7c on a tongue 7a of a user 7, i.e., the first oral cavity stimulation electrode 21 is disposed at a back of the tongue, the second oral cavity stimulation electrode 22 is disposed at the middle of the tongue, and the first oral cavity stimulation electrode 21a is disposed at a tip of the tongue. Thus, in the present embodiment, as the pair of electrodes, there are three electrodes respectively disposed at the tip of the tongue, the middle of the tongue, and the back of the tongue, and a pair is formed of the second oral cavity stimulation electrode 22 at the middle of the tongue and the first oral cavity stimulation electrodes (21, 21a) at the tip of the tongue and at the back of the tongue. Moreover, skin stimulation electrodes (32a, 32b) are disposed to be branched to right and left, on a skin of a neck 7b of the user 7. The skin stimulation electrodes (32a, 32b) are electrodes branched from the first oral cavity stimulation electrodes (21, 21a) and are disposed on the skin surface. The skin stimulation electrodes (32a, 32b) and the first oral cavity stimulation electrodes (21, 21a) have an equivalent electric potential. The skin stimulation electrodes (32a, 32b) and the second oral cavity stimulation electrode 22 have opposite potentials to each other, and the first oral cavity stimulation electrodes (21, 21a) and the second oral cavity stimulation electrode 22 have opposite potentials to each other.

Thus, three electrodes are provided on the tongue and the first oral cavity stimulation electrodes (21, 21a) are disposed so as to sandwich the second oral cavity stimulation electrode 22, and thereby a current density directly under the first oral cavity stimulation electrode 21 and the second oral cavity stimulation electrode 22 or the first oral cavity stimulation electrode 21a and the second oral cavity stimulation electrode 22 increases, and therefore the oral muscles can be effectively trained in a long range corresponding to an electrode length of three electrodes. By disposing the skin stimulation electrodes (32a, 32b) to be branched to right and left, a wide range of the oral muscles can be trained.

### Embodiment 9

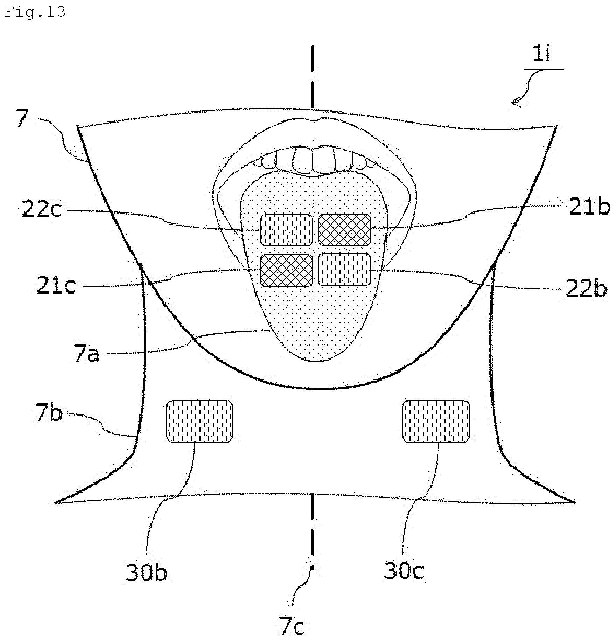

Fig. 13 shows an image diagram of electrode disposition according to another embodiment. As shown in Fig. 13, in an oral muscle training device 1i, first oral cavity stimulation electrodes (21b, 21c) and second oral cavity stimulation electrodes (22b, 22c) are disposed on a tongue 7a of a user 7. The first oral cavity stimulation electrode 21b and the first oral cavity stimulation electrode 21c have an equivalent electric potential, and the second oral cavity stimulation electrode 22b, the second oral cavity stimulation electrode 22c, the skin stimulation electrode 30b, and the skin stimulation electrode 30c have an equivalent electric potential. The first oral cavity stimulation electrodes (21b, 21c) and the second oral cavity stimulation electrodes (22b, 22c) have opposite potentials to each other and the first oral cavity stimulation electrodes (21b, 21c) and the skin stimulation electrodes (30b, 30c) have opposite potentials to each other. Namely, the first oral cavity stimulation electrodes (21b, 21c) and the second oral cavity stimulation electrodes (22b, 22c) are disposed so that adjacent electrodes have opposite polarities to each other.

The pair of electrodes in the present embodiment is formed of the first oral cavity stimulation electrodes (21b, 21c), which are a plurality of electrodes, and the second oral cavity stimulation electrodes (22b, 22c), which are a plurality of electrodes, and the adjacent electrodes have opposite polarities to each other. Moreover, skin stimulation electrodes (30b, 30c) are disposed to be branched to right and left, on a skin of a neck 7b of the user 7.

Thus, by disposing four electrodes on the tongue and the adjacent poles have opposite polarities to each other, a current density directly under the first oral cavity stimulation electrode 21b and the second oral cavity stimulation electrode 22b, the first oral cavity stimulation electrode 21b and the second oral cavity stimulation electrode 22c, the first oral cavity stimulation electrode 21c and the second oral cavity stimulation electrode 22b, or the first oral cavity stimulation electrode 21c and the second oral cavity stimulation electrode 22c increases when applying the current to the electrodes, and therefore the oral muscle can be effectively trained in a wider range corresponding to the four electrodes. Moreover, the oral muscles can easily be trained selectively by disposing the skin stimulation electrodes (30b, 30c).

### Embodiment 10

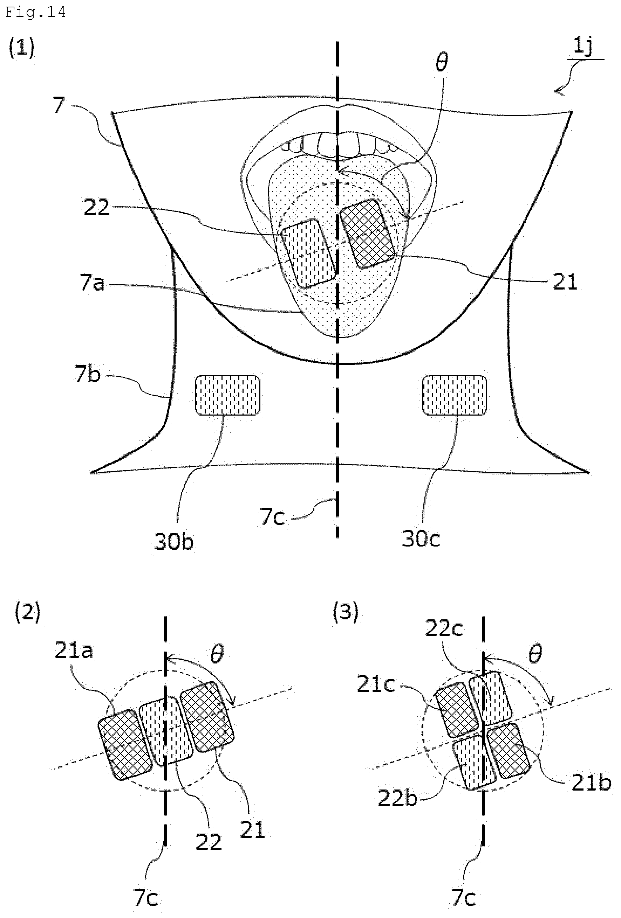

Fig. 14 is image diagram of electrode disposition in still further another embodiment, in which Fig. 14(1) shows a case of two electrodes, Fig. 14(2) shows a case of three electrodes, and Fig. 14(3) shows a case of four electrodes, each disposed on a tongue. An oral muscle training device 1j is obtained by rotating the electrode disposition state of any one of the oral muscle training devices (1f, 1h, 1i) within a range of 180°. In the example shown in Fig. 14(1), a first oral cavity stimulation electrode 21 disposed at a back of the tongue and a second oral cavity stimulation electrode 22 disposed at a tip of the tongue is in a state of being rotated within a range of 180°. By setting an angle θ, an area under the tongue where a current density is high can be changed. Moreover, in the example shown in Fig. 14(2), a first oral cavity stimulation electrode 21 disposed at a back of the tongue, a second oral cavity stimulation electrode 22 disposed at the middle of the tongue, and a first oral cavity stimulation electrode 21a disposed at a tip of the tongue are in a state of being rotated within a range of 180°. Moreover, in the example shown in Fig. 14(3), first oral cavity stimulation electrodes (21b, 21c) and second oral cavity stimulation electrodes (22b, 22c), which are disposed so that adjacent electrodes have opposite polarities to each other, are in a state of being rotated within a range of 180°. Also in this case, by setting an angle θ, an area under the tongue where a current density is high can be changed.

### Embodiment 11

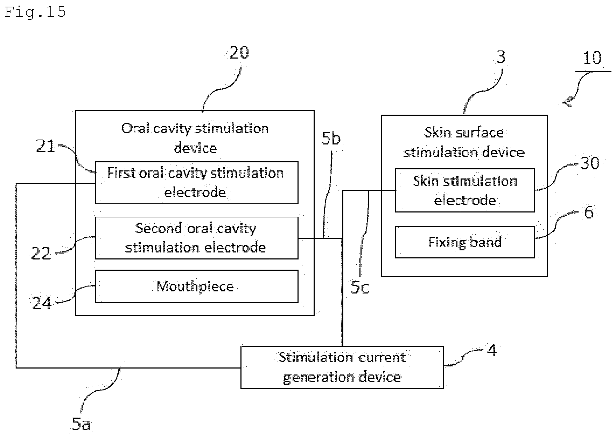

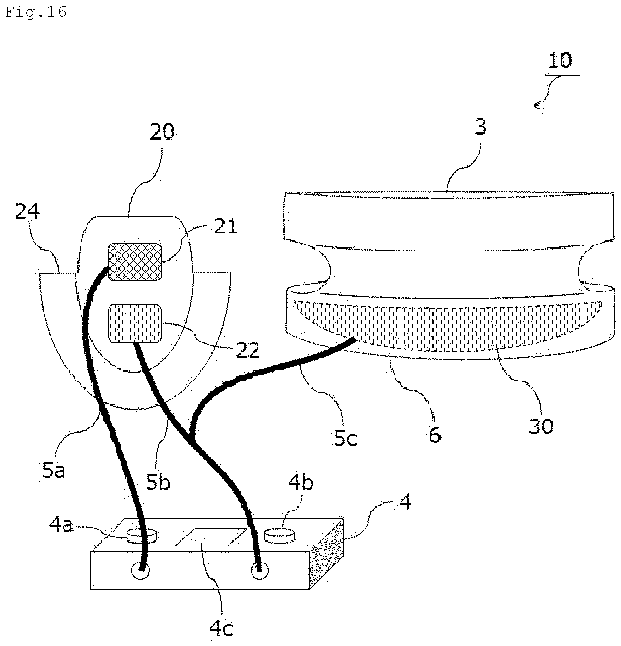

Fig. 15 shows a functional block diagram of an oral muscle training device according to another embodiment. Fig. 16 shows a configuration image diagram of the oral muscle training device. As shown in Fig. 15, the oral muscle training device 10 includes an oral cavity stimulation device 20, a skin surface stimulation device 3, and a stimulation current generation device 4. The oral cavity stimulation device 20 includes a first oral cavity stimulation electrode 21 and a second oral cavity stimulation electrode 22 as a pair of electrodes disposed in an oral cavity, and a mouthpiece 24.

As shown in Fig. 16, the first oral cavity stimulation electrode 21 and the second oral cavity stimulation electrode 22 are attached to be fixed to the mouthpiece 24. The rest of the configurations is the same as that of the oral muscle training device 1 according to the embodiment 1. Since the first oral cavity stimulation electrode 21 and the second oral cavity stimulation electrode 22 are fixed to the mouthpiece 24, a user can fix the oral cavity stimulation electrodes (21, 22) to an appropriate position by merely wearing the mouthpiece 24, thereby improving training effect and convenience.

### Embodiment 12

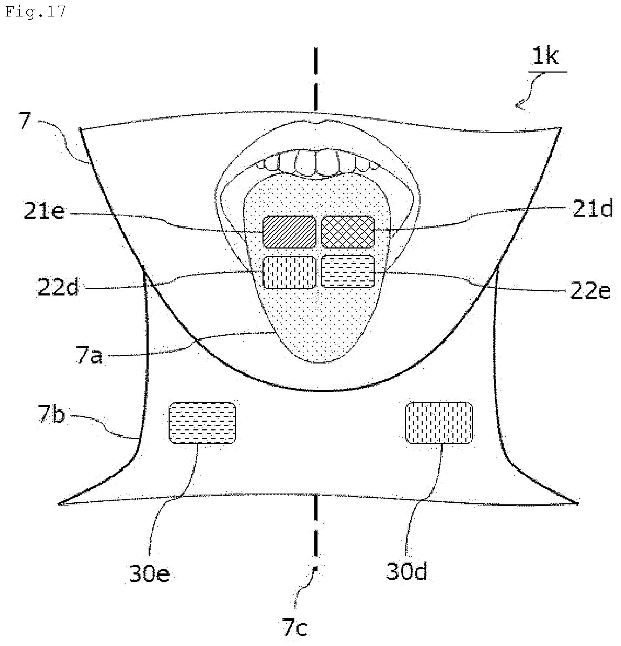

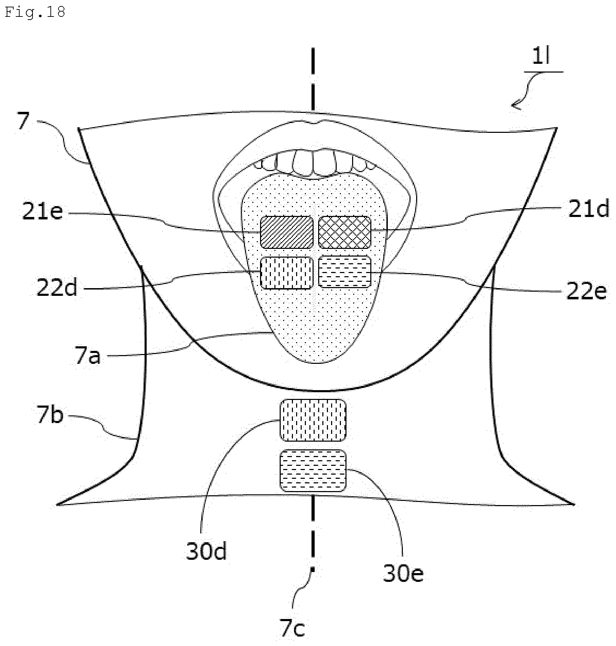

Fig. 17 shows an image diagram of electrode disposition in the present embodiment. Fig. 18 shows an image diagram of electrode disposition in another embodiment. It is to be noted that, for convenience of explanation, in Figs. 17 and 18, only a disposition image of electrodes in the device is illustrated, and shape images of the electrodes are deformed.

An oral muscle training device 1k according to a present embodiment includes: a first pair of electrodes (21d, 22d) disposed on a tongue; a first surface electrode (skin stimulation electrode 30d) branched from one electrode (22d) of the first pair of electrodes and is disposed on a front surface of a neck; an electric circuit configured to pass a first medium frequency current between electrodes formed of the first pair of electrodes (21d, 22d) and the first surface electrode (30d); similarly, a second pair of electrodes (21e, 22e) disposed on the tongue; a second surface electrode (skin stimulation electrode 30e) branched from one electrode (22e) of the second pair of electrodes and is disposed on the front surface of the neck; and an electric circuit configured to pass a second medium frequency current between electrodes formed of the second pair of electrodes (21e, 22e) and the second surface electrode (30e). The skin stimulation electrodes (30d, 30e), which are first and second surface electrodes are disposed in line symmetry with respect to a midline 7c.

In other words, the oral muscle training device 1k is formed of: a first channel including a first pair of electrodes (21d, 22d) disposed on a tongue 7a of a user 7, a first surface electrode (skin stimulation electrode 30d) branched from one electrode (22d) of the first pair of electrodes and is disposed on a front surface of a neck, an electric circuit configured to pass a first medium frequency current (e.g., 5050 Hz) between electrodes formed of the first pair of electrodes (21d, 22d) and the first surface electrode (30d); and a second channel including a second pair of electrodes (21e, 22e) disposed on the tongue 7a of the user 7, a second surface electrode (skin stimulation electrode 30e) branched from one electrode (22e) of the second pair of electrodes and is disposed on the front surface of the neck, and an electric circuit configured to pass a second medium frequency current (e.g., 5000 Hz) between electrodes formed of the second pair of electrodes (21e, 22e) and the second surface electrode (30e).

For example, when the first medium frequency that flows through the first channel is 5050 Hz, and the second medium frequency that flows through the second channel is 5000 Hz, an interference wave is generated due to a frequency difference of 50 Hz and a large amount of current flows between the electrodes in the oral cavity and the surface electrode on the front surface of the neck, the oral muscles, such as the genioglossus muscle, can be stimulated using this interference wave. The first medium frequency current and the second medium frequency current may have the same frequency, in which case no interference wave is generated.

In the present embodiment, the first surface electrode (skin stimulation electrode 30d) is branched from the one electrode (22d) of the first pair of electrodes (21d, 22d), but may be branched from the other electrode (21d) thereof. Similarly, the second surface electrode (skin stimulation electrode 30e) is branched from the one electrode (22e) of the second pair of electrodes (21e, 22e), but may be branched from the other electrode (21e) thereof. Moreover, the skin stimulation electrodes (30d, 30e), which are first and second surface electrodes, are disposed in line symmetry with respect to the midline 7c, but their positions may be reversed right and left, or they may be disposed in a vertical direction on the midline 7c as shown in Fig. 18. By disposing the skin stimulation electrode (30d, 30e) in the vertical direction on the midline 7c, central deep muscles can be stimulated more effectively. In addition, the skin stimulation electrode (30d, 30e) may be disposed upside down on the midline 7c or may be disposed under the chin.

The locations of the first pair of electrodes (21d, 22d) and the second pair of electrodes (21e, 22e) can be interchanged between the tip of the tongue and the back of the tongue, respectively.

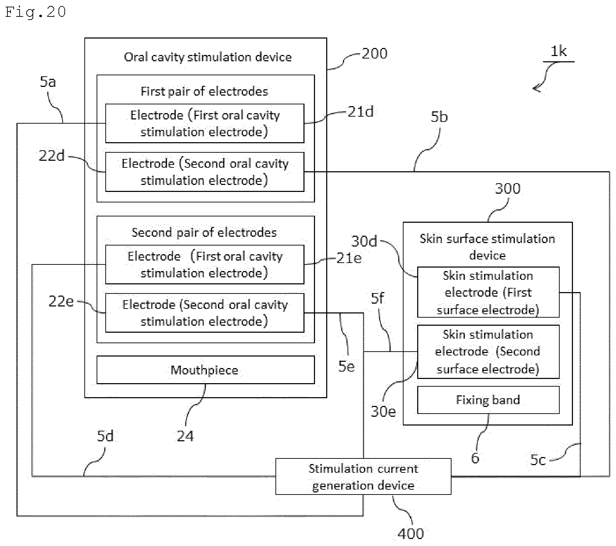

Fig. 20 shows a functional block diagram of the oral muscle training device according to the present embodiment. As shown in Fig. 20, the oral muscle training device 1k includes an oral cavity stimulation device 200, a skin surface stimulation device 300, and a stimulation current generation device 400. The oral cavity stimulation device 200 includes a first pair of electrodes (21d, 22d), a second pair of electrodes (21e, 22e), and a mouthpiece 24. The skin surface stimulation device 300 includes skin stimulation electrodes (30d, 30e) and a fixing band 6. The stimulation current generation device 400 is connected to the electrode 21d through a lead wire 5a and is connected to the electrode 22d through a lead wire 5b. The skin stimulation electrode 30d is branched from the electrode 22d using a lead wire 5c and is connected to the stimulation current generation device 400.

Moreover, the stimulation current generation device 400 is connected to the electrode 21e through a lead wire 5d and is connected to the electrode 22e through a lead wire 5e. The skin stimulation electrode 30e is branched from the electrode 22e using a lead wire 5f and is connected to the stimulation current generation device 400.

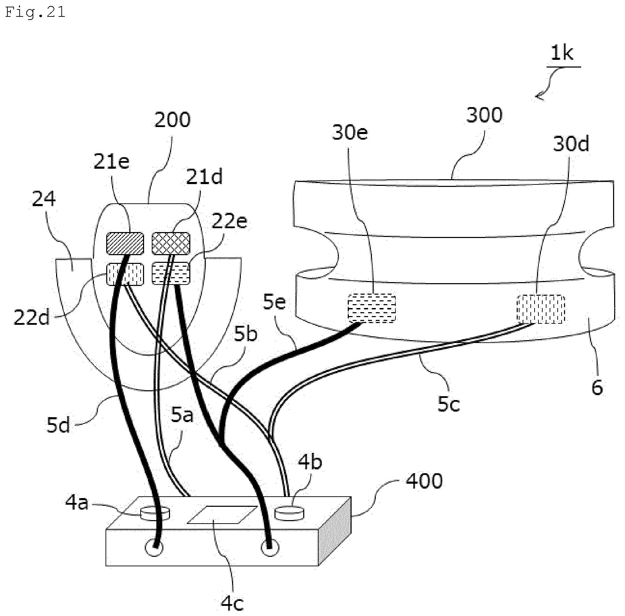

Fig. 21 shows a configuration image diagram of the oral muscle training device according to the present embodiment. As shown in Fig. 21, the first pair of electrodes (21d, 22d) and the second pair of electrodes (21e, 22e) are attached to be fixed to the mouthpiece 24, and the skin stimulation electrodes (30d, 30e) are attached to be fixed to the fixing band 6. By fixing the electrodes to the mouthpiece 24 and the fixing band 6, the user can provide and fix the electrodes at an appropriate position merely by wearing the mouthpiece 24 and the fixing band 6, thereby improving a training effect and convenience.

Operation buttons (4a, 4b, and other buttons not shown) are provided for switching the first medium frequency current ON/OFF, for adjusting the frequency of the first medium frequency current, for switching the second medium frequency current ON/OFF, and for adjusting the frequency of the second medium frequency current.

The difference in illustration between the lead wires (5a to 5c) and the lead wires (5d to 5f) in Fig. 21 is to clearly distinguish between the first channel and the second channel, and both are made of the same material.

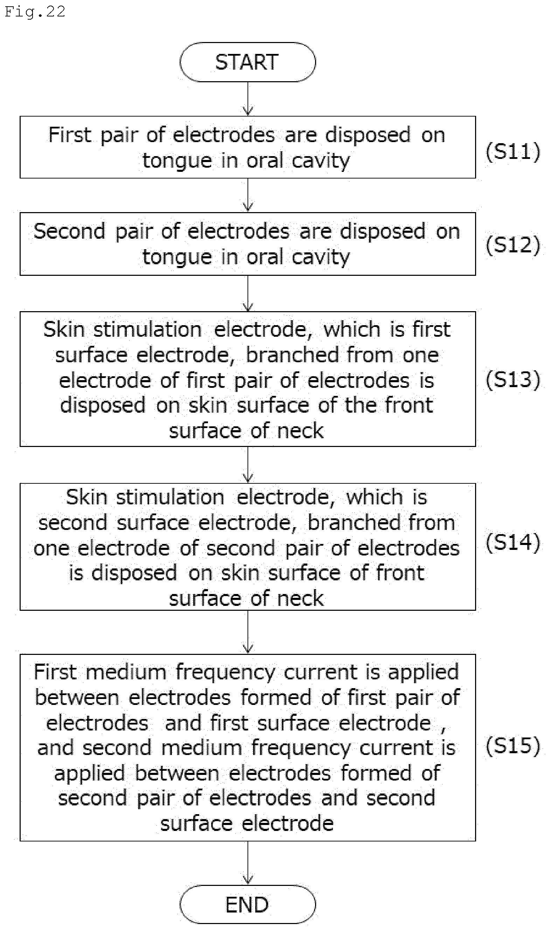

Fig. 22 shows a schematic flow chart of an oral muscle training method according to the present embodiment. As shown in Fig. 22, first, the first pair of electrodes (21d, 22d) are disposed on the tongue in the oral cavity (Step S11), and the second pair of electrodes (21e, 22e) are disposed on the tongue in the oral cavity (Step S12). It is to be noted that when the first pair of electrodes (21d, 22d) and the second pair of electrodes (21e, 22e) are attached to the mouthpiece 24, the processes of Steps S11 and S12 are simultaneously performed.

Subsequently, the skin stimulation electrode (30d), which is the first surface electrode, branched from the one electrode of the first pair of electrodes (21d, 22d) is disposed on the skin surface of the front surface of the neck (Step S13), and the skin stimulation electrode (30e), which is the second surface electrode, branched from the one electrode of the second pair of electrodes (21e, 22e) is disposed on the skin surface of the front surface of the neck (Step S14). It is to be noted that when the skin stimulation electrodes (30d, 30e) are fixed by the fixing band 6, the processes of Steps S13 and S14 are simultaneously performed.

Then, the first medium frequency current is applied between the electrodes formed of the first pair of electrodes (21d, 22d) and the first surface electrode (30d), and the second medium frequency current having a frequency slightly different from that of the first medium frequency current is applied between the electrodes formed of the second pair of electrodes (21e, 22e) and the second surface electrode (30e) (Step S15). It is to be noted that the order of Steps S11 to S14 may be changed.

### Embodiment 13

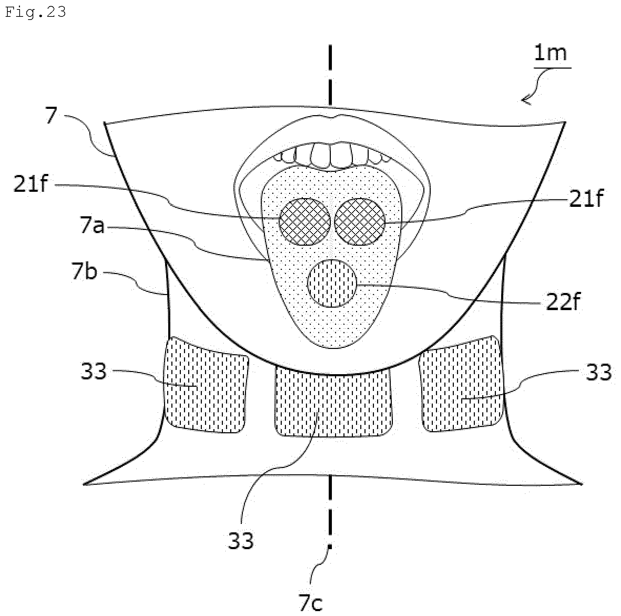

Fig. 23 shows an image diagram of electrode disposition in the present embodiment. As shown in Fig. 23, in an oral muscle training device 1m, on a tongue 7a of a user 7, two first oral cavity stimulation electrodes 21f are disposed in parallel so as to straddle a midline 7c, and the second oral cavity stimulation electrode 22f is disposed along the midline 7c. The first oral cavity stimulation electrode 21f is disposed at a back of the tongue, and the second oral cavity stimulation electrode 22f is disposed at a tip of the tongue. The two first oral cavity stimulation electrodes 21f are arranged side by side in order to increase a size of the first oral cavity stimulation electrode. Accordingly, one large-sized first oral cavity stimulation electrode may be disposed as in the embodiment 14 described below.

Moreover, the skin stimulation electrode 33 is disposed on the skin of the neck 7b of the user 7 so as to cover substantially an entire periphery of the neck 7b. In Fig. 23, the three skin stimulation electrodes 33 are disposed at approximately equal intervals on the front side of the neck, but actually, other three skin stimulation electrodes 33 are disposed at approximately equal intervals also on a back surface side of the neck in a similar manner, covering substantially an entire periphery of the neck 7b as a whole. By providing the skin stimulation electrode 30 around substantially the entire periphery of the neck 7b, it is possible to pass the medium frequency current over a wide area to be stimulated. Moreover, a current density of the electrode disposed on the skin surface can be reduced, preventing blood flow in the carotid arteries from being inhibited.

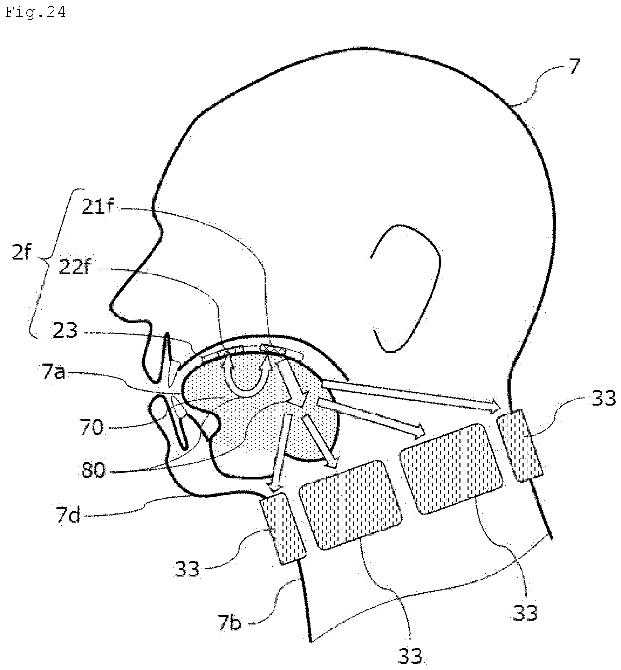

Fig. 24 shows a functional explanatory diagram of an oral muscle training device according to the present embodiment. As shown in Fig. 24, in an oral cavity stimulation device 2f, a positive or negative electrode is disposed on the tongue 7a of the user 7, and an electrode branched from the one electrode is disposed at a position of the neck, which is a position sandwiching oral muscles, such as a tongue muscle and a genioglossus muscle; and as shown in an image 80 of a current flow, the oral muscles such as the tongue muscle and the genioglossus muscle 70 are stimulated with a current flowing through the first oral cavity stimulation electrode 21, the second oral cavity stimulation electrode 22, and the skin stimulation electrode 33.

A current measurement test (N=2) is conducted using the electrode disposition in the embodiment 13. The following Tables 2 and 3 show respectively voltage, current, and resistance values of the oral cavity stimulation electrode or the skin stimulation electrode when the current of each frequency is applied. The Table 2 below shows a current measurement test result for a test subject A, and the Table 3 below shows a current measurement test result for a test subject B. The applied current is a continuous wave current between 4 kHz to 8 kHz. The item "sensation" means qualitative intensity of the stimulation felt by the test subjects A and B during the test, expressed quantitatively on a 5-point scale from 0 to 4.

**[Table 2]**

| Frequency (Hz) | Oral cavity stimulation electrode | | | Skin stimulation electrode | | | Sensation |
|---|---|---|---|---|---|---|---|
| | Voltage (V) | Current (mA) | Resistance (Q) | Voltage (V) | Current (mA) | Resistance (Ω) | |
| 4000Hz | 2.6 | **4.8** | 541.7 | 2.6 | **10.2** | 254.9 | 2 |
| 6000Hz | 1.7 | **6.0** | 283.3 | 1.7 | **9.0** | 188.9 | 0-1 |
| 8000Hz | 1.6 | **5.6** | 285.7 | 1.6 | **9.5** | 168.4 | 1 |

**[Table 3]**

| Frequency (Hz) | Oral cavity stimulation electrode | | | Skin stimulation electrode | | | Sensation |
|---|---|---|---|---|---|---|---|
| | Voltage (V) | Current (mA) | Resistance (Ω) | Voltage (V) | Current (mA) | Resistance (Ω) | |
| 4000Hz | 3.4 | **6.1** | 557.4 | 3.4 | **8.0** | 425.0 | 3 |
| 6000Hz | 1.8 | **6.8** | 264.7 | 1.8 | **7.8** | 230.8 | 1 |
| 8000Hz | 1.3 | **6.6** | 197.0 | 1.3 | **7.8** | 166.7 | 1 |

As shown in Table 2, for the test subject A, the current values flowing through the electrodes in the oral cavity are 4.8 mA at 4000 Hz, 6.0 mA at 6000 Hz, and 5.6 mA at 8000 Hz. In contrast, the current values flowing through the electrodes on the skin of the neck are 10.2 mA at 4000 Hz, 9.0 mA at 6000 Hz, and 9.5 mA at 8000 Hz, indicating that the current flowing through the skin stimulation electrode is greater. It is found that the test subject A felt the most stimulation at 4000 Hz.

As shown in Table 3, for the test subject B, the current values flowing through the electrodes in the oral cavity are 6.1 mA at 4000 Hz, 6.8 mA at 6000 Hz, and 6.6 mA at 8000 Hz. In contrast, the current values flowing through the electrodes on the skin of the neck are 8.0 mA at 4000 Hz, 7.8 mA at 6000 Hz, and 7.8 mA at 8000 Hz, indicating that the current flowing through the skin stimulation electrode is greater. It is found that the test subject B felt the most stimulation at 4000 Hz.

Since electrodes in the oral cavity are highly uncomfortable, electrical stimulation must also be performed with as little stimulation discomfort as possible. In the present test, it is found that the frequency with the least muscle stimulation and discomfort is 4000 Hz.

### Embodiment 14

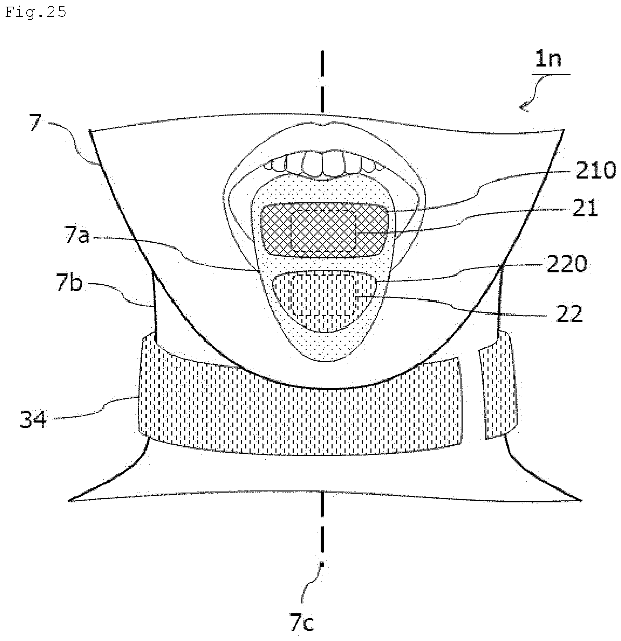

Fig. 25 shows an image diagram of electrode disposition in the present embodiment. As shown in Fig. 25, in an oral muscle training device 1n, a first oral cavity stimulation electrode 210 and a second oral cavity stimulation electrode 220 are disposed along a midline 7c on a tongue 7a of a user 7, i.e., the first oral cavity stimulation electrode 210 is disposed at a tip of the tongue, and the second oral cavity stimulation electrode 220 is disposed at a back of the tongue. Unlike the first oral cavity stimulation electrode 21 according to the embodiment 1, the first oral cavity stimulation electrode 210 according to the present embodiment is provided larger within a range where a vomiting center is not stimulated and a predetermined distance to the second oral cavity stimulation electrodes 220 can be provided. Moreover, similarly, unlike the second oral cavity stimulation electrode 22 according to the embodiment 1, the second oral cavity stimulation electrode 220 is provided larger within a range where a predetermined distance to the first oral cavity stimulation electrodes 210 can provided.

Moreover, the skin stimulation electrode 34 may be disposed on a skin of a neck 7b of the user 7 so as to cover substantially an entire periphery of the neck 7b. Unlike the skin stimulation electrode 33, the skin stimulation electrodes 34 as one electrode is configured to be wrapped around substantially an entire periphery of the neck 7b. Consequently, the medium frequency current can be applied over a large area to be stimulated. Moreover, a current density of the electrode disposed on the skin surface can be reduced, preventing blood flow in the carotid arteries from being inhibited.

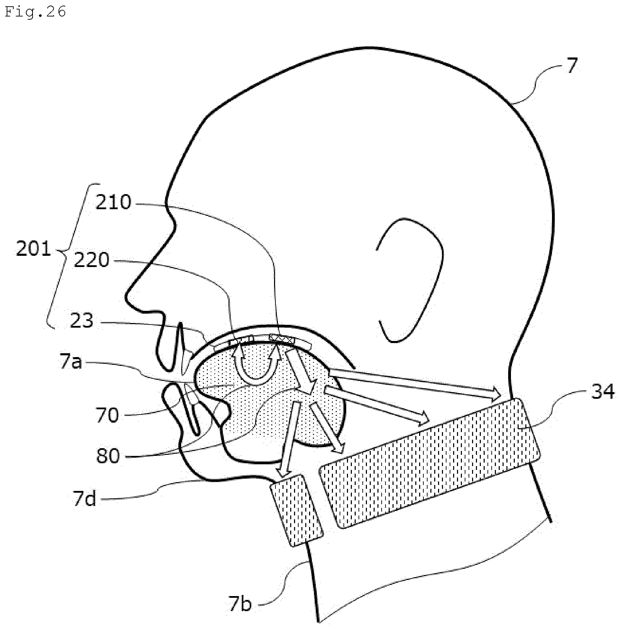

Fig. 26 shows a functional explanatory diagram of the oral muscle training device according to the present embodiment. As shown in Fig. 26, in an oral cavity stimulation device 201, a positive or negative electrode is disposed on the tongue 7a of the user 7, and an electrode branched from the one electrode is disposed at a position of the neck, which is a position sandwiching oral muscles, such as a tongue muscle and a genioglossus muscle; and as shown in an image 80 of a current flow, the oral muscles such as the tongue muscle and the genioglossus muscle 70 are stimulated with a current flowing through the first oral cavity stimulation electrode 21, the second oral cavity stimulation electrode 22, and the skin stimulation electrode 34.

### Other Embodiments

In the oral muscle training devices of the above-described embodiments, the continuous wave current is used as the medium frequency current, but a burst wave current may be used in which a current equal to or more than 3 kHz and less than 8 kHz, more preferably, equal to or more than 3 kHz and less than 5 kHz, flows continuously for a predetermined period of time and then is repeatedly stopped for a predetermined time (e.g., one second or more). By intermittently passing the medium frequency current, it is possible to avoid a situation where the tongue is pulled and fixed toward a front tooth side. Here, a state where no current flows for a predetermined period of time (intermittent state) may mean a state in which no current flows at all for one second or more, or a state in which the medium frequency current is used as a low frequency intermittent wave of several Hz. It has been empirically found that a medium frequency current intermittency for two seconds or more is preferable since it is possible to swallow saliva.

Generally, it has been known that 20 to 50 Hz leads to muscular hypertrophy of fast muscles and 4 to 8 Hz leads to muscular hypertrophy of slow muscles. On the other hand, medium frequencies have characteristics of low skin resistance and being easy to reach deep areas, but it is difficult to provide effective stimulation leading to muscle hypertrophy. Therefore, in order to stimulate deep genioglossus muscles, medium frequencies are used, but to use of medium frequency waves as intermittent waves of 4 to 8 Hz may stimulate slow muscles, and to use of medium frequency waves as intermittent waves of 20 to 50 Hz may stimulate fast muscles. Furthermore, intermittent use of medium frequency as a low frequency of 100 Hz has a potential for nerve regeneration.

### Industrial Applicability

The present invention can effectively stimulate the genioglossus muscle, which is a deep muscle in the oral cavity, and is useful as a sleep apnea treatment device using electric stimulation. The present invention is also useful for swallowing training using electrical stimulation by adjusting the size, current amount, and frequency of the electrodes to stimulate not only the genioglossus muscle but also the geniohyoid muscle.

### Reference Signs List

1, 1a to 1n, 10: Oral muscle training device
2, 20, 200, 201: Oral cavity stimulation device
3, 300: Skin surface stimulation device
4, 400: Stimulation current generation device
4a, 4b: Operation button
4c: Display unit
5a to 5f: Lead wire
6: Fixing band
7: User
7a: Tongue
7b: Neck
7c: Midline
7d: Under chin
8, 80: (current flow) Image
21, 21a to 21e, 210: First oral cavity stimulation electrode
22, 22b to 22e, 220: Second oral cavity stimulation electrode
23: Base
24: Mouthpiece
30, 30a to 30e, 31, 32a, 32b, 33, 34: Skin stimulation electrode
70: Genioglossus muscle

## Claims

1. An oral muscle training device comprising:
a pair of electrodes disposed in an oral cavity;
an electrode branched from one electrode of the pair of electrodes and disposed on a skin surface; and
an electric circuit configured to pass a medium frequency current between the electrodes.

2. The oral muscle training device according to claim 1, wherein the pair of electrodes are disposed on a tongue, and
the electrode disposed on the skin surface is disposed under a chin or on a front surface of a neck.

3. The oral muscle training device according to claim 1, wherein the pair of electrodes are disposed on a tongue, and
the electrode disposed on the skin surface is disposed around approximately an entire periphery of a neck.

4. The oral muscle training device according to claim 2 or **3,** wherein the pair of electrodes are respectively disposed at a tip of the tongue and a back of the tongue.

5. The oral muscle training device according to claim 2 or **3,** wherein the pair of electrodes are disposed on a left side or a right side of a midline, and the electrode disposed on the skin surface is disposed on an opposite side of the pair of electrodes across the midline.

6. The oral muscle training device according to claim 2 or **3,** wherein the pair of electrodes are disposed in point symmetry with respect to a point on a center line of the tongue between a tip of the tongue and a back of the tongue.

7. The oral muscle training device according to claim 2, wherein the electrode disposed on the skin surface is branched into at least two electrodes, and when there is an even number of branched electrodes, the electrodes are disposed in line symmetry with respect to a midline, or when there is an odd number of branched electrodes, one electrode is disposed on the midline and the rest of the electrodes is disposed in line symmetry with respect to the midline.

8. The oral muscle training device according to claim 2 or **3,** wherein there are three electrodes respectively disposed at a tip of the tongue, a middle of the tongue, and a back of the tongue, and a pair is formed of the electrodes disposed at the middle and tip of the tongue and another pair is formed of the electrodes disposed at the middle and back of the tongue.

9. The oral muscle training device according to claim 2 or **3,** wherein a plurality of pairs of electrodes are disposed as the pair of electrodes, in which adjacent electrodes are opposite poles to each other.

10. The oral muscle training device according to claim 1, wherein the medium frequency current is a continuous wave current equal to or more than 3 kHz and less than 8 kHz.

11. The oral muscle training device according to claim 1, wherein the medium frequency current is an intermittent wave in which a current equal to or more than 3 kHz and less than 8 kHz flows continuously for a predetermined period of time and then does not flow for a predetermined period of time, or an intermittent wave current having a low frequency of 1 to 100 Hz used as a current equal to or more than 3 kHz and less than 8 kHz.

12. An oral muscle training device comprising:
a first pair of electrodes disposed in an oral cavity; a first surface electrode branched from one electrode of the first pair of electrodes and disposed on a skin surface; an electric circuit configured to pass a first medium frequency current between electrodes formed of the first pair of electrodes and the first surface electrode;
a second pair of electrodes disposed in the oral cavity; a second surface electrode branched from one electrode of the second pair of electrodes and disposed on the skin surface; and an electric circuit configured to pass a second medium frequency current between electrodes formed of the second pair of electrodes and the second surface electrode.

13. The oral muscle training device according to claim 12, wherein the first and second pair of electrodes are disposed on a tongue, and
the first and second surface electrodes are disposed under a chin or on a front surface of a neck.

14. A set of the electrodes disposed in the oral cavity and the electrode disposed on the skin surface in the oral muscle training device according to any one of claims 1-3, 12, and 13.

15. An oral muscle training method comprising:
disposing a pair of electrodes on a tongue in an oral cavity;
disposing an electrode branched from one electrode of the pair of electrodes under a chin or on a skin surface of a front surface of a neck; and
passing a medium frequency current between the electrodes.

16. An oral muscle training method comprising:
disposing a pair of electrodes on a tongue in an oral cavity;
disposing an electrode branched from one electrode of the pair of electrodes around substantially an entire periphery of the neck; and
passing a medium frequency current between the electrodes.

17. An oral muscle training method comprising:
disposing a first pair of electrodes on a tongue in an oral cavity;
disposing a second pair of electrodes on the tongue in the oral cavity;
disposing a first surface electrode branched from one electrode of the first pair of electrodes under a chin or on a skin surface of a front surface of a neck;
disposing a second surface electrode branched from one electrode of the second pair of electrodes under the chin or on the skin surface of the front surface of the neck; and
passing a first medium frequency current between electrodes formed of the first pair of electrodes and the first surface electrode and a second medium frequency current between electrodes formed of the second pair of electrodes and the second surface electrode.
